(19) Europäisches Patentamt
European Patent Office
Office européen des brevets

(11) Veröffentlichungsnummer: **0 386 452 B1**

(12) **EUROPÄISCHE PATENTSCHRIFT**

(45) Veröffentlichungstag der Patentschrift: **22.09.93**

(21) Anmeldenummer: **90101944.8**

(22) Anmeldetag: **01.02.90**

Teilanmeldung 93104118.0 eingereicht am 01/02/90.

(51) Int. Cl.5: **C07C 323/56**, C07C 323/16, C07C 323/62, C07C 323/19, C07C 323/36, C07C 317/22, C07C 317/44, C07C 235/42, C07C 229/38, C07C 229/60, C07C 259/10

# (54) Diphenylheteroalkylderivate, ihre Herstellung und daraus hergestellte Arzneimittel und Kosmetika.

(30) Priorität: **10.02.89 DE 3903989**

(43) Veröffentlichungstag der Anmeldung:
**12.09.90 Patentblatt 90/37**

(45) Bekanntmachung des Hinweises auf die Patenterteilung:
**22.09.93 Patentblatt 93/38**

(84) Benannte Vertragsstaaten:
**AT BE CH DE DK FR GB IT LI NL SE**

(56) Entgegenhaltungen:
**EP-A- 0 288 305**
**EP-A- 0 317 165**
**DE-A- 1 911 539**
**FR-A- 2 413 382**

(73) Patentinhaber: **BASF Aktiengesellschaft**
**Carl-Bosch-Strasse 38**
**D-67063 Ludwigshafen(DE)**

(72) Erfinder: **Janssen, Bernd, Dr.**
**Leuschnerstrasse 18 a**
**D-6700 Ludwigshafen(DE)**
Erfinder: **Wuest, Hans-Heiner, Dr.**
**Unteres Bieth 10**
**D-6915 Dossenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Es ist bekannt, daß Stilbenderivate [vgl. DE-OS 2 854 354, DE-OS 3 534 564 und EP 212-137 (US 4 588 750)], bei welchen die Polyenstruktur von Substanzen des vitamin-A-Typs in aromatischen Ringen fixiert vorliegt, pharmakologische Wirkungen bei der topischen und systemischen Therapie von Neoplasien, Akne, Psoriasis und anderen dermatologischen Affektionen aufweisen. Die Wirkung dieser Verbindungen befriedigt jedoch nicht immer [vgl. G.L. Peck in: The Retinoids, Vol. II, 391-409, Ed.: M.B. Sporn et al., Academic Press N.Y. (1984), oder R. Marks et al., Med. J. Australia 146, 374-377 (1987), oder C.E. Orfanos et al., Drugs 34, 459-503 (1987)].

Der Erfindung lag daher die Aufgabe zugrunde, Verbindungen mit besserem Wirkspektrum zu entwikkeln.

Überraschend wurde nun gefunden, daß Diphenylheteroalkylderivate der Formel I

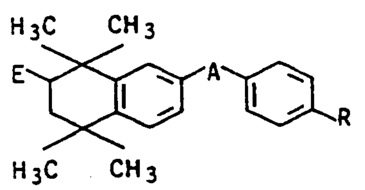

(I)

in der bedeuten:

A eine $-X-CH_2-$ oder

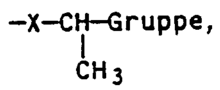

wobei X mit dem linken oder dem rechten Phenylkern verknüpft sein kann und für ein Sauerstoffatom, eine $-S(O)_n-$ oder -NR'-Gruppe steht (mit n in der Bedeutung von 0, 1 oder 2 und R' in der Bedeutung von Wasserstoff, $C_{1-6}$-Alkyl oder $C_{1-6}$-Alkanoyl),

E Wasserstoff oder Hydroxyl und

R eine der Gruppen $-CO_2H$, $-CO_2-C_{1-6}$-Alkyl, -CHO, $-CH_2OH$, -CN, $-CONH_2$, $-CH_2NH_2$, $-NH-C_{1-4}$-Alkanoyl, $-N(C_{1-4}\text{-Alkyl})_2$, $-S(O)_n-C_{1-4}$-Alkyl (mit n = 0, 1 oder 2),

sowie gegebenenfalls deren physiologisch verträglichen Salze ein besseres Wirkspektrum besitzen.

Von den Verbindungen der Formel I sind diejenigen bevorzugt, in denen A die oben angegebene Bedeutung hat und X für eine Ether-, Thioether- oder NH-Brücke steht.

Die neuen Verbindungen der Formel I enthalten teilweise chirale Zentren und fallen im allgemeinen als Diastereomerengemische, bzw. Racemate an. Die so erhaltenen Diasteromeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) werden von der vorliegenden Erfindung umfaßt. Als therapeutische oder kosmetische Mittel kann man sowohl die einheitlichen Diastereomeren bzw. Enantiomeren wie auch deren Gemische verwenden.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium- und Alkalimetallsalze, insbesondere des Natriums, Kaliums und Lithiums, oder Erdalkalimetallsalze, insbesondere des Calciums oder Magnesiums, sowie Salze mit geeigneten organischen Basen, wie mit niederen Alkylaminen, z. B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure übergeführt. Als übliche physiologisch verträgliche organische oder anorganische Säure kommen beispielsweise in Betracht: Salzsäure, Bromwasserstoffsäure, Phosphorsäure oder Schwefelsäure und als organische Säuren beispielsweise Maleinsäure, Fumarsäure, Milchsäure, Weinsäure, Äpfelsäure, Zitronensäure, Salicylsäure, Adipinsäure

oder Benzoesäure. Weitere können aus "Fortschritte der Arnzeimittelforschung" Band 10, Seiten 224-225, Birkhäuser Verlag, Basel und Stuttgart, 1966, entnommen werden.

Gegenstand der vorliegenden Erfindung ist weiter ein Verfahren zur Herstellung der oben angegebenen Verbindungen der Formel I, indem man

a) - falls A eine $-O-CH_2-$ oder $-O-CH(CH_3)-$Gruppe bedeutet - Phenole der Formel II

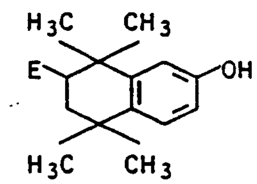

(II),

mit Benzylderivaten der Formel III

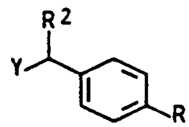

(III),

in der $R^2$ ein Wasserstoffatom oder eine Methylgruppe und Y eine nukleofuge Abgangsgruppe darstellt, in Gegenwart einer Base umsetzt, oder

b) - falls A eine $-CH_2-O-$ oder $-CH(CH_3)-O-$Gruppe bedeutet - Phenole der Formel IV

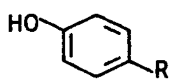

(IV),

mit Benzylderivaten der Formel V

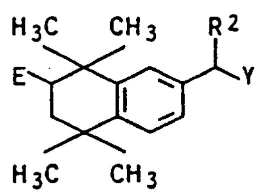

(V),

in der E, $R^2$ und Y jeweils die genannten Bedeutungen haben, in Gegenwart einer Base umsetzt, oder

c) - falls A eine $-S(O)_n-CH_2-$ oder $-S(O)_n-CH(CH_3)-$Gruppe bedeutet - Thiophenole der Formel VI

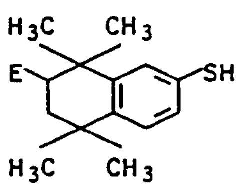

(VI),

mit Benzylderivaten der Formel III wie unter a) angegeben zu Thioethern (n = 0) umsetzt und diese gegebenenfalls mit einem Oxidationsmittel in die korrespondierenden Sulfoxide (n = 1) oder Sulfone (n = 2) überführt, oder

d) - falls A eine $-CH_2-S(O)_n-$ oder $-CH(CH_3)-S(O)_n-$Gruppe bedeutet -Thiophenole der Formel VII

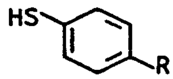

(VII),

in der R die in Anspruch 1 angegebene Bedeutung hat, mit Benzylderivaten der Formel V wie unter b) angegeben zu Thioethern (n = 0) umsetzt und diese gegebenenfalls mit einem Oxidationsmittel in die korrespondierenden Sulfoxide (n = 1) oder Sulfone (n = 2) überführt, oder

e) - falls A eine $-NR'CH_2-$ oder $-NR'CH(CH_3)-$Gruppe bedeutet - entweder

Anilinderivate der Formel VIII

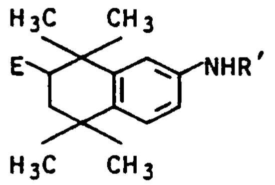

(VIII),

mit Benzylderivaten der Formel III wie unter a) angegeben umsetzt, oder

Anilinderivate der Formel VIII mit Carbonylverbindungen der Formel IX

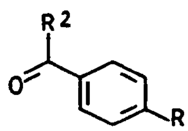

(IX),

in der R und $R^2$ die genannten Bedeutungen haben, in Gegenwart eines Reduktionsmittels umsetzt, oder

f) - falls A eine $-CH_2NR'-$ oder $-CH(CH_3)NR'-$Gruppe bedeutet - entweder

1. Anilinderivate der Formel X

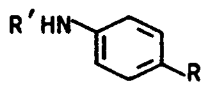

(X),

mit Benzylderivaten der Formel V wie unter b) angegeben umsetzt, oder

2. Aniline der Formel X mit Carbonylverbindungen der Formel XI

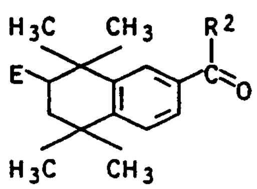

(XI),

in Gegenwart eines Reduktionsmittels umsetzt

und die so erhaltenen Verbindungen gegebenenfalls nach Standardmethoden in weitere Verbindungen der Formel I und gegebenenfalls ihre physiologisch verträglichen Salze umwandelt.

Die Alkylierungsreaktionen a)-d) sowie e1) und f1) erfolgen in an sich bekannter Weise, gegebenenfalls in Gegenwart eines Lösungs- oder verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Als nucleofuge Abgangsgruppen kommen vorzugsweise in Betracht: Brom, Chlor, die Methylsulfonyloxy-, Trifluormethylsulfonyloxy- und die Toluolsulfonyloxygruppe.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat, Ryridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-tri-ethylammoniumchlorid oder -bromid oder Kronenether wie 12-Krone-4, 15-Krone-5, 18-Krone-6, Di-benzo-18-Krone-6 oder Dicyclohexano-18-Krone-6 in Frage.

Die Umsetzung wird im allgemeinen bei Temperaturen zwischen 20 und 150 °C, drucklos oder unter Druck, kontinuierlich oder diskontinuierlich durchgeführt.

Die unter c) und d) angeführten Oxidationsreaktionen der Thioether erfolgen gegebenenfalls in Gegenwart eines Lösungs- oder Verdünnungsmittels und gegebenenfalls unter Zusatz eines Katalysators, indem man 1.0 Äquivalent des Thioethers mit 1.0 bis 1.1 Äquivalenten des Oxidationsmittels bei Temperaturen von -30 bis 120 °C zu dem entsprechenden Sulfoxid umsetzt.

Die Darstellung der entsprechenden Sulfone erfolgt durch Verwendung von 2.0 bis 3.0 Äquivalenten an Oxidationsmittel. Zu den bevorzugten Lösungs- oder Verdünnungsmitteln gehören niedere Alkylcarbonsäuren wie Ameisensäure, Essigsäure und Propionsäure, Alkohole wie Methanol, Ethanol oder Isopropanol, Kohlenwasserstoffe wie Hexan, Cyclohexan oder Heptan, Aromaten wie Benzol, Toluol oder Xylol, Ether wie Methyl-tert.-butylether, Diisopropylether oder Diphenylether, Ketone wie Aceton oder Methylethylketon, halogenierte Kohlenwasserstoffe wie Dichlormethan, Chloroform, Tetrachlorkohlenstoff, Dichlorethan oder Chlorbenzol, aber auch Nitrile wie Acetonitril und Propionitril oder Amide wie Dimethylformamid, Dimethylacetamid oder Pyrrolidon sowie Wasser; es sind aber auch Gemische derselben geeignet.

Als Oxidationsmittel kommen in Frage:
Peroxiverbindungen wie Wasserstoffperoxid, tert.Butylhydroperoxid, Peressigsäure, Perbenzoesäure, mono-Perphthalsäure oder halogenierte Perbenzoesäuren wie m-Chlorperbenzoesäure. Es sind aber auch andere Oxidationsmittel wie Kaliumpermanganat, Kaliumdichromat, Natriumperjodat oder Perjodsäure sowie Salpetersäure oder Nitrosegase wie Stickstoffdioxid verwendbar (vgl. beispielsweise "Methoden der Organischen Chemie" Hrsg. Eugen Müller, Bd. IX, S. 207 ff und S. 223 ff, Thieme Verlag, Stuttgart 1955, sowie Reid, "Organic Compounds of Bivalent Sulfur", Bd. 2, S. 64 ff, Chem. Publ. New York, 1960).

Als Katalysatoren kommen gegebenenfalls in Betracht Mineralsäuren wie Chlorwasserstoffsäure oder Schwefelsäure, aber auch Alkalihydroxide wie Natrium- oder Kaliumhydroxid sowie Alkalicarbonate wie Natrium- oder Kaliumcarbonat.

Im Falle, daß die Reaktion in einem zweiphasigen System abläuft, können Phasentransferkatalysatoren, z. B. quarternäre Ammoniumverbindungen wie Tetrabutylammoniumsalze, zur Reaktionsbeschleunigung verwendet werden.

Die Alkylierungsreaktionen e2) und f2) erfolgen in an sich bekannter Weise im Sinne einer reduktiven Alkylierung, gegebenenfalls in Gegenwart eines Lösungs- oder Verdünnungsmittels, gegebenenfalls unter Zusatz eines Reaktionsbeschleunigers. Die Umsetzung wird im allgemeinen bei Temperaturen zwischen 20 und 150 °C, drucklos oder unter Druck, kontinuierlich oder diskontinuierlich durchgeführt.

Die Anilin- und die Carbonylkomponente werden im Verhältnis 0.5:1 bis 2:1, vorzugsweise in äquimolarem Verhältnis eingesetzt.

Zu den bevorzugten Lösungsmitteln gehören Kohlenwasserstoffe wie Heptan, Cyclohexan, Toluol oder Xylol, ferner Ether wie Diethylether, Tetrahydrofuran oder Dioxan, Halogenkohlenwasserstoffe wie Dichlormethan, Trichlormethan oder Chlorbenzol, Alkohole wie Methanol, Ethanol, Isopropanol oder Cyclohexanol, Alkyl-Carbonsäuren wie Ameisensäure, Essigsäure oder Propionsäure, ferner Acetonitril, Wasser oder entsprechende Gemische.

Als Reduktionsmittel kommen in Frage, Ameisensäure, Wasserstoff oder Metallhydride wie Lithiumaluminiumhydrid, Natriumborhydrid oder Natriumcyanoborhydrid.

Es sind aber auch andere Reduktionsmittel einsetzbar [vgl. "Methoden der Organischen Chemie", Hrsg. Eugen Müller, Bd. XI/1, S. 618 ff, S. 648 ff, S. 669 ff, Thieme Verlag, Stuttgart 1957, oder Watanabe et al. Tetrahedron Lett. 1879 (1974)].

Als Hydrierkatalysatoren in homogener oder heterogener Phase kommen bevorzugt in Betracht Nickel, Cobalt oder Platinkatalysatoren (vgl. Marko et al., J. Organomet. Chem. 81, 411 (1974), bzw. Rylander, "Catalytic Hydrogenation over Platinum Metals", S. 291-303, Academic Press, N.Y., 1967).

Die Ausgangsverbindungen der Formel II sind bekannt oder können nach den allgemein üblichen Methoden zur Herstellung substituierter Phenolderivate erhalten werden ("Methoden der Organischen Chemie", Hrsg. Eugen Müller, Bd. VI/1c, S. 4 ff, S. 313 ff, S. 925 ff, Thieme Verlag, Stuttgart, 1976).

Die Benzylderivate der Formel III, worin Y für ein Brom- oder Chloratom steht, sind bekannt oder lassen sich darstellen aus den entsprechenden Alkylbenzolderivaten (Y = Wasserstoff) durch Halogenierung in an sich bekannter Weise ("Methoden der Organischen Chemie", Hrsg. Eugen Müller, Bd. V/3, S. 735 ff, S. 809, Thieme Verlag, Stuttgart, 1962 und Bd. V/4, S. 219 ff, Thieme Verlag, Stuttgart, 1960; W. Foerst: "Neuere Methoden der präparat. org. Chemie", Bd. III, S. 134, Verlag Chemie, Weinheim, 1961).

Benzylderivate der Formel III, worin Y für eine OH-Gruppe steht, sind teilweise bekannt oder werden hergestellt durch Reduktion der korrespondierenden Carbonylderivate der Formel IX. Die Darstellung der reaktiven Ester der Formel III, worin Y eine nucleofuge Abgangsgruppe bedeutet, erfolgt hieraus nach allgemein bekannten Methoden (Houben-Weyl-Muller, Methoden der organischen Chemie, Georg Thieme Verlag, Stuttgart, 1955, Band IX, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Bromobenzolsulfonsäureester, 4-Nitrobenzolsulfonsäureester oder Benzolsulfonsäureester.

Die Ausgangsverbindungen der Formel IV sind bekannt oder werden nach allgemein bekannten Verfahren zur Herstellung substituierter Phenole erhalten, entsprechend den zur Herstellung von Verbindungen des Typs II oben angegebenen Methoden.

Die Benzylderivate der Formel V, worin Y für ein Brom- oder Chloratom steht, sind bekannt. Sie lassen sich herstellen aus den entsprechenden Alkylbenzolderivaten (Y = Wasserstoff) durch Halogenierung in an sich bekannter Weise (s. Literaturhinweis bei Ausgangsstoffen der Formel III).

Verbindungen der Formel V, worin Y eine reaktive Estergruppe bedeutet, werden entsprechend dem für die Vorstufen der Formel III oben angegebenen Weg erhalten, indem man die korrespondierenden Carbonylverbindungen der Formel XI reduziert und anschließend in die reaktiven Ester überführt.

Die Ausgangsverbindungen der Formel VI sind bekannt oder lassen sich nach den für die Darstellung von Thiophenolen üblichen Verfahren erhalten (K.-D. Gundermann und K. Hümke in "Methoden der Organischen Chemie", Bd. E11, S. 32 ff, Thieme Verlag, Stuttgart, 1985 und dort zitierte Literatur). Sie lassen sich bevorzugt herstellen durch Reduktion der entsprechenden Sulfonsäurederivate, zum Beispiel mit Metallhydriden, oder auch aus den entsprechenden Phenolen der Formel II, die zu Thiocarbaminsäureestern umgesetzt werden (Newman u. Karnes, J. Org. Chem. 31, 3980 (1966)).

Die Ausgangsverbindungen der Formel VII sind bekannt oder lassen sich nach allgemein üblichen Verfahren - wie für die Thiophenole der Formel VI oben beschrieben - herstellen.

Die Ausgangsverbindungen der Formel VIII sind bekannt oder werden in an sich bekannter Weise hergestellt, beispielsweise durch Reduktion der entsprechenden Nitroverbindungen ("Methoden der Organischen Chemie", Hrsg. E. Müller, Bd. XI/1, S. 394, Thieme-Verlag, Stuttgart, 1957).

Die Ausgangsverbindungen der Formel IX sind bekannt oder werden erhalten nach üblichen Methoden zur Darstellung von Acetophenonen, beispielsweise durch Friedel-Crafts Acylierung (H.O. House: "Modern Synthetic Reactions", 2nd Ed., W.A. Benjamin Inc. Menlo Park, CA, (1972), S. 797 ff und dort zitierte Literatur) oder durch Oxidation der entsprechenden Alkylbenzole (H.O. House. l.c., S. 288 f und dort angegebene Literatur) sowie zur Darstellung von Benzaldehyden, beispielsweise durch Aromatenformylierung nach Vilsmeier (vgl. De. Meheas, Bull. Soc. Chem. Fr. 1989-1999 (1962) und dort zitierte Literatur) oder durch Reduktion der entsprechenden Benzoylhalogenide (vgl. Fuson in: Patai, "The Chemistry of the Carbonyl Group", Vol. 1, S. 211- 232, Interscience Publ., N.Y. 1966 oder Wheeler in: Patai, "The Chemistry of Acyl Halides" S. 231-251, Interscience Publ. N.Y. 1972) oder Benzonitrile (vgl. J. March: "Advanced Organic Chemistry, 2nd Ed., McGraw-Hill Kogakusha LTD. Tokyo, 1977, S. 835-836 und dort zitierte Literatur).

Die Ausgangsverbindungen der Formel X sind bekannt oder werden hergestellt nach üblichen Methoden analog den oben bereits für die Darstellung von Anilinen des Typs VIII angegebenen.

Die Ausgangsverbindungen der Formel XI sind bekannt (z.B.: DE-OS 3 602 473, DE-OS 3 434 942, DE-OS 3 434 944) oder werden entsprechend den oben für die Herstellung von Carbonylverbindungen der Struktur IV angegebenen Verfahren erhalten.

Die erfindungsgemäßen Verbindungen der Formel I, in der A eine NR'-Gruppe, mit R' in der Bedeutung von Wasserstoff, enthält, werden nach üblichen Methoden zur N-Alkylierung oder N-Acylierung in an sich bekannter Weise zu weiteren erfindungsgemäßen Verbindungen der Formel I umgesetzt.

Die nach den oben genannten Verfahren a-f hergestellten Substanzen lassen sich anschließend wie folgt weiter abwandeln:

Die Benzoesäureester der Formel I ( R = Carboalkoxy) werden, falls erwünscht, durch Esterverseifung in die freien Carbonsäuren übergeführt. Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispeilsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder Isopropanol, oder in Dimethylsulfoxid, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol, Methanol und Dimethylsulfoxid, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niedere Trialkylaminen, wie Trimethyl- oder Triethylamin, in Gemischen mit Wasser. Dabei wird die verwendete Base im Verhältnis zum Ester in stöchiometrischer Menge oder in geringem überschuß eingesetzt. Vorzugsweise wird Natrium- oder Kaliumhydroxid verwendet.

Die Veresterung geschieht vorteilhaft, indem man die Carbonsäure zunächst in ihr Salz überführt und dieses mit einem entsprechenden Alkylhalogenid, vorzugsweise einem Alkylbromid oder -iodid, behandelt. Als Deprotonierungsmittel für die Herstellung der Salze in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid und insbesondere Methylethylketon, wobei die Umsetzung beim Siedepunkt des Reaktionsgemisches durchgeführt wird.

Die erfindungsgemäßen Amide können in an sich bekannter Weise hergestellt werden durch Amidolyse der Ester.

Eine Carbonsäure, ein Carbonsäureester oder ein Carbonsäureamid der Formel I kann in an sich bekannter Weise zu dem entsprechenden Alkohol bzw. Amin reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion mit Diisobutylaluminiumhydrid oder einem Alkoxynatriumaluminiumhydrid durch, so ist die Verwendung von Kohlenwasserstoffen wie Hexan oder Toluol bevorzugt.

So erhaltene Amine oder Alkohole können in an sich bekannter Weise mit einem Alkanoylhalogenid oder -anhydrid oder einem Aroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs-oder Lösungsmittel in die erfindungsgemäßen Amide und Ester der Formel (I) überführt werden. Bevorzugt werden die Umsetzungen in Gegenwart einer Base als säurebindendem Mittel in einem zwischen -20 °C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Acylierungsmittel in katalytischer Menge oder in stöchiometrischer Menge bzw. in geringem überschuß verwendet werden.

Ein unter die Formel I fallender Alkohol kann mit geeigneten Oxidationsmitteln, vorzugsweise Mangan-(IV)-oxid, gegebenenfallS auf einem anorganischen Trägermaterial wie Silicagel oder Aluminiumoxid zum entsprechenden Aldehyd oxidiert werden. Vorteilhaft arbeitet man in einem inerten organischen Lösungsmittel, beispielsweise einem Kohlenwasserstoff wie Hexan oder in einem Ether wie Tetrahydrofuran oder in Mischungen der genannten Lösungs- und Verdünnungsmittel im Temperaturbereich zwischen -10 °C und 30 °C. Die benötigte Reaktionszeit ist im wesentlichen von der Oxidationsaktivität des eingesetzten Mangan-(IV)-oxids abhängig.

Einen Aldehyd I (R = -CHO) kann man auch durch Reduktion des entsprechenden Nitrils mit Diisobutylaluminiumhydrid in einem Lösungsmittel vorzugsweise in Toluol, Hexan, Tetrahydrofuran oder Mischungen dieser Lösungsmittel in einem Temperaturbereich zwischen -40 °C und Raumtemperatur erhalten.

Die unter die Formel I fallenden Aldehyde und Ketone werden auch dadurch erhalten, daß man ihre Ketale hydrolysiert, üblicherweise in Gegenwart einer Säure als Katalysator, vorzugsweise verdünnter Salz- oder Schwefelsäure, bei 20 °C oder bei bis zum Siedepunkt des Reaktionsgemisches erhöhter Temperatur. Zweckmäßigerweise arbeitet man in mit Wasser gemischten Lösungsmitteln wie Aceton, Dioxan, Tetrahydrofuran, vorzugsweise in kurzkettigen Alkoholen wie Methanol und Ethanol.

Ein Nitril der Formel I (R = -CN) kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie Methanol, Ethanol, Isopropanol oder n-Butanol eingesetzt. Die Umsetzung wird üblicherweise beim Siedepunkt des Reaktionsgemisches durchgeführt.

Die erfindungsgemäßen Verbindungen und ihre physiologisch verträglichen Salze können aufgrund ihrer pharmakologischen Eigenschaften bei der topischen und systemischen Therapie und auch Prophylaxe von

Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, Ekzeme, aber auch gegen Vitiligo, Warzen, Lichtschäden (vorzeitige Alterung) der Haut, ferner gegen trockene Augen und andere Corneopathien sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Bevorzugte Indikationsgebiete sind: die Therapie von dermatologischen Erkrankungen; die Therapie von durch Sonnenlichteinwirkung bedingten Hautschädigungen; die Therapie von iatrogen, z.B. durch Corticosteroide induzierte Atrophie, bedingten Hautschädigungen und die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben.

Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J.Am. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung therapeutische Mittel zur topischen und systemischen Anwendung sowie kosmetische Mittel, die eine Verbindung der Formel (I) als Wirkstoff neben üblichen Trägerstoffen oder Verdünnungsmitteln enthalten.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Zweckmäßige übliche Bestandteile von kosmetischen und pharmazeutischen Zubereitungen für die topische Anwendung sind beispielsweise:
anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Triglyceridester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, etherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Die nachfolgenden Beispiele erläutern die Erfindung:

Herstellung der Ausgangsverbindungen

Beispiel A

5,6,7,8-Tetrahydro-5,5,8,8-tetramethyl-2-naphthol

250,0 g (2,65 mol) Phenol wurden mit 414,5 g (2,27 mol) 2,5-Dichlor-2,5-dimethylhexan in 500 ml Petrolether bei Raumtemperatur unter Rühren mit 27,5 g (0,2 mol) wasserfreiem Aluminiumchlorid spatelweise versetzt. Nach 48 Stunden wurde das Reaktionsgemisch auf Eiswasser gegossen, mit Ether extrahiert und die organische Phase mit Wasser neutral gewaschen, über Natriumsulfat getrocknet und im Vakuum eingedampft. Der Rückstand ergab nach zweifachem Umkristallisieren aus Methanol 148,7 g der Titelverbindung, Schmp. 219-220 °C.

Beispiel B

5,6,7,8-Tetrahydro-5,5,8,8-tetramethyl-2-naphthylamin

Zu einer Lösung von 65,8 g (0,35 mol) 1,2,3,4-Tetrahydro-1,1,4,4-tetramethylnaphthalin in 154 ml Eisessig und 257 ml Essigsäureanhydrid wurde im Eis-Kochsalzbad ein unter Kühlen bereitetes Gemisch aus 77,2 ml Eisessig und 20,8 ml Salpetersäure (98 %ig) binnen 2 Stunden zugetropft. Nach beendeter Zugabe wurde das Reaktionsgemisch auf Raumtemperatur erwärmt und über Nacht gerührt. Die Lösung wurde danach auf Wasser gegossen, der Niederschlag abgesaugt, mit Wasser nachgewaschen und getrocknet. Man erhielt 79,7 g rohes 2-Nitro-5,6,7,8-tetrahydro-5,5,8,8-tetramethylnaphthalin vom Schmp. 46-50 °C.

25 g dieses Rohproduktes wurden in einem Gemisch aus 65 ml Dioxan, 65 ml Methanol und 5 ml Wasser über 0,3 g Palladium auf Aktivkohle (10 %) während 48 Stunden bei 100 °C und 200 bar Wasserstoff im Autoklaven hydriert. Nach beendeter Reaktion wurde vom Katalysator abfiltriert und die Lösung eingedampft. Umkristallisieren des Rückstandes aus n-Heptan lieferte 17,6 g der Titelverbindung, Schmp. 63-65 °C.

Beispiel C

5,6,7,8-Tetrahydro-5,5,8,8-tetramethyl-2-thionaphthol

94,0 g (0,5 mol) 1,2,3,4-Tetrahydro-1,1,4,4-tetramethylnaphthalin wurden binnen 30 min bei 20 °C in 100 ml Chlorsulfonsäure eingerührt. Die Reaktionslösung blieb 1 Stunde bei 60 °C, wurde nach dem Abkühlen auf Raumtemperatur auf 1,5 l Eis gegossen und mit Ether extrahiert. Die organische Phase wurde mit wäßriger Kochsalzlösung und Wasser neutral gewaschen, über Magnesiumsulfat getrocknet und im Vakuum

eingedampft. Umkristallisieren des Rückstandes aus Methanol ergab 55,0 g 5,6,7,8-Tetrahydro-5,5,8,8-tetramethyl-naphthalin-2-sulfonsäurechlorid, Schmp. 71-74 °C.

57,5 g (0,2 mol) des so hergestellten Sulfonsäurechlorids wurden in 150 ml trockenem Tetrahydrofuran binnen 2 Stunden bei Raumtemperatur zu einer Suspension aus 15,4 g (0,4 mol) Lithiumaluminiumhydrid in 150 ml trockenem Tetrahydrofuran getropft. Es wurde 1 Stunde bei Raumtemperatur nachgerührt, die Reaktionslösung tropfenweise mit 25 ml Wasser, danach mit 50 ml gesättigter Weinsäurelösung versetzt und wenige Minuten zum Sieden erhitzt. Nach dem Abkühlen wurde die Lösung mit wasserfreiem Magnesiumsulfat bis zum Aufklaren versetzt und vom Niederschlag abgesaugt.

Eindampfen des Filtrats erbrachte 33,0 g der Titelverbindung, die als Harz anfiel ($R_F$ = 0,4, Heptan/Essigester = 7:3).

Herstellung der Endprodukte

Beispiel 1

(4-Cyanobenzyl)-(5,6,7,8-tetrahydro-5,5,8,8-tetramethyl-2-naphthalenyl)ether

40,8 g (0,2 mol) 5,6,7,8-Tetrahydro-5,5,8,8-tetramethyl-2-naphthyl, 30,4 g (0,2 mol) 4-Cyanobenzylchlorid und 80 g (1,2 mol) wasserfreies Kaliumcarbonat wurden in 400 ml Butanon und 300 ml Dimethylformamid 9 h unter Rückfluß erhitzt. Nach dem Abkühlen goß man auf 1,5 l Wasser, saugte den gebildeten Feststoff ab und wusch ihn mit Wasser nach. Nach Trocknen erhielt man 63 g der Titelverbindung, Schmp. 149-150 °C.

Beispiel 2

(4-Carboxybenzyl)-(5,6,7,8-tetrahydro-5,5,8,8-tetramethyl-2-naphthalenyl)ether

20 g (0,063 mol) (4-Cyanobenzyl)-(5,6,7,8-tetrahydro-5,5,8,8-tetramethyl-2-naphthalenyl)ether wurden mit 163 ml 10 N Natronlauge in 245 ml Ethanol 2 h unter Rückfluß erhitzt. Nach dem Abkühlen goß man auf Wasser, säuerte mit Salzsäure an und saugte den ausgefallenen Feststoff ab, der mit Wasser und Methanol gewaschen wurde. Nach dem Trocknen erhielt man 19,7 g der Titelverbindung, Schmp. >330 °C.

Beispiel 3

(4-Formylbenzyl)-(5,6,7,8-tetrahydro-5,5,8,8-tetramethyl-2-naphthalenyl)ether

Zu einer Lösung von 3 g (9,4 mmol) (4-Cyanobenzyl)-(5,6,7,8-tetrahydro-5,5,8,8-tetramethyl-2-naphthalenyl)ether in 40 ml trockenem Ether wurden unter Stickstoff bei 25°C 16,5 ml (19,7 mmol) Diisobutylaluminiumhydridlösung (20 % in Hexan) zugetropft. Man ließ 40 min bei 25 °C rühren und tropfte dann 250 ml ges. Weinsäurelösung zu. Man gab anschließend etwas Natriumsulfatlösung zu und trennte die Phasen. Die wäßrige Phase wurde mit Ether extrahiert, der organische Extrakt mit ges. Weinsäurelösung und Wasser gewaschen, über $Na_2SO_4$ getrocknet und eingeengt. Nach Umkristallisation aus Methanol erhielt man 1,4 g der Titelverbindung, Schmp. 102-104 °C.

Beispiel 4

(4-Carboxamidobenzyl)-5,6,7,8-tetrahydro-5,5,8,8-tetramethyl-2-naphthalenyl)ether

2 g (6,3 mmol) (4-Cyanobenzyl)-(5,6,7,8-tetrahydro-5,5,8,8-tetramethyl-2-naphtalenyl)ether und 5,9 g Kaliumhydroxidpulver wurden in 47,5 ml tert.-Butanol 45 min unter Rückfluß erhitzt. Mach dem Abkühlen wurde das Reaktionsgemisch auf ges. Natriumchloridlösung gegossen und mit Ether extrahiert. Der in der Etherphase ausgefallene Niederschlag wurde abgesaugt und aus Isopropanol umkristallisiert. Man erhielt so 1,3 g der Titelverbindung, Schmp. 202-205 °C. Aus der Etherphase wurden nach üblicher Aufarbeitung weitere 0,5 g des Produktes gewonnen.

Beispiel 5

(4-Aminomethylbenzyl)-(5,6,7,8-tetrahydro-5,5,8,8-tetramethyl-2-naphthalenyl)ether

Zu einer Suspension von 1 g (26 mmol) Lithiumaluminiumhydrid in 50 ml trockenem Ether wurde unter Stickstoff und bei 25 ° C eine Suspension von 3 g (9,4 mmol) (4-Cyanobenzyl)-(5,6,7,8-tetra-hydro-5,5,8,8-tetramethyl-2-naphthalenyl)ether in 60 ml Ether zugetropft. Danach ließ man 3 h unter Rückfluß rühren. Nach dem Abkühlen wurde der Ansatz vorsichtig mit Wasser und Natriumsulfatlösung hydrolysiert. Man extrahierte dreimal mit Ether, wusch die vereinigten Etherextrakte mit Wasser, trocknete über $Na_2SO_4$ und engte ein. Es verblieben 3 g der Titelverbindung, Schmp. 77-79 ° C.

Beispiel 6

(4-Hydroxymethylbenzyl)-(5,6,7,8-tetrahydro-5,5,8,8-tetramethyl-2-naphthalenyl)ether

Zu einer Lösung von 3 g (9 mmol) (4-Carboxybenzyl)-(5,6,7,8-tetrahydro-5,5,8,8-tetramethyl-2-naphthalenyl)ether und 0,9 g (9 mmol) Triethylamin in 20 ml trockenem Tetrahydrofuran wurde bei 0 ° C eine Lösung von 0,97 g (9 mmol) Chlorameisensäureethylester in 5 ml Tetrahydrofuran zugetropft. Man ließ 30 min bei 0 ° C nachrühren, filtrierte vom Feststoff ab und tropfte das Filtrat zu einer Lösung von 0,83 g (22 mmol) Natriumborhydrid in 8 ml Tetrahydrofuran und 8 ml Wasser. Danach ließ man das Reaktionsgemisch binnen 1 h auf Raumtemperatur kommen und säuerte dann mit 1 N Salzsäure an. Das Tetrahydrofuran wurde weitgehend am Rotationsverdampfer im Vakuum entfernt und die verbliebene wäßrige Phase mit Chloroform extrahiert. Die organische Phase wurde über $Na_2SO_4$ getrocknet und eingeengt. Durch fraktionierte Kristallisation aus Essigester, bei der die erste Fraktion verworfen wurde, erhielt man 1,1 g der Titelverbindung, Schmp. 108-109 ° C.

Beispiel 7

(4-Carbethoxybenzyl)-(5,6,7,8-tetrahydro-5,5,8,8-tetramethyl-2-naphthalenyl)ether

2 g (6 mmol) (4-Carboxybenzyl)-(5,6,7,8-tetrahydro-5,5,8,8-tetramethyl-2-naphthalenyl)ether, 2,7 g (19,6 mmol) wasserfreies Kaliumcarbonat und 19 g (12,4 mmol) Iodethan wurden in 18 ml Butanon 7 h unter Rückfluß erhitzt. Danach goß man auf Wasser, extrahierte mit Ether, wusch die organische Phase mit Wasser, trocknete über $Na_2SO_4$ und engte ein. Nach umkristallisation aus Methanol erhielt man 0,9 g der Titelverbindung, Schmp. 76-77 ° C.

Beispiel 8

(4-Carbethoxyphenyl)-(5,6,7,8-tetrahydro-5,5,8,8-tetramethyl-2-naphthalenyl)methylether

10 g (36 mmol) 2-Brommethyl-5,6,7,8-tetrahydro-5,5,8,8-tetramethylnaphthalin, 6 g (36 mmol) 4-Hydroxybenzoesäureethylester und 7,2 g (52 mmol) wasserfreies Kaliumcarbonat wurden in 60 ml Dimethylformamid 5,25 h unter Rückfluß erhitzt. Danach goß man auf Wasser, extrahierte mehrmals mit Ether, wusch die organische Phase mit Wasser, trocknete über $Na_2SO_4$ und engte ein. Nach Umkristalllsation aus Methanol erhielt man 6,7 g der Titelverbindung, Schmp. 109-11 ° C.

Beipiel 9

(4-Carboxyphenyl)-(5,6,7,8-tetrahydro-5,5,8,8-tetramethyl-2-naphthalenyl)methylether

Analog Beispiel 2 erhielt man aus 3 g (8,5 mmol) (4-Carbethoxyphenyl)-(5,6,7,8-tetrahydro-5,5,8,8-tetramethyl-2-naphthalenyl)methylether 2,6 g der Titelverbindung, Schmp. 185-186 ° C.

Beispiel 10

(4-Carboxybenzyl)-(5,6,7,8-tetrahydro-5,5,8,8-tetramethyl-2-naphthalenyl)thioether

6,6 g (30 mmol) 5,6,7,8-Tetrahydro-5,5,8,8-tetramethyl-2-thionaphthol und 3,5 g (30 mmol) Kalium-tert.-butanolat wurden in 80 ml Dimethylformamid bei Raumtemperatur mit 5,7 g (26 mmol) 4-Brommethylbenzoesäure in 20 ml Dimethylformamid versetzt. Nach Rühren über Nacht bei Raumtemperatur wurde die Reaktionslösung auf Wasser gegossen und mit Essigester extrahiert. Der organische Extrakt wurde mit Wasser gewaschen, über Magnesiumsulfat getrocknet und im Vakuum eingedampft. Der Rückstand lieferte nach Umkristallisieren aus Cyclohexan 4,0 g der Titelverbindung, Schmp.169-170 °C.

Beispiel 11

N-(4-Carboxybenzyl)-(5,6,7,8-tetrahydro-5,5,8,8-tetramethyl-2-naphthalenyl)amin

8,1 g (40 mmol) 5,6,7,8-Tetrahydro-5,5,8,8-tetramethyl-2-naphthylamin wurden mit 6,6 g (40 mmol) 4-Formylbenzoesäuremethylester in 100 ml Toluol 30 min unter Rückfluß erhitzt, das Lösungsmittel wurde abgedampft, der Rückstand in 200 ml Tetrahydrofuran/Methanol (1:1) aufgenommen und bei Raumtemperatur mit 3,2 g (50 mmol) Natriumcyanoborhydrid versetzt. Nach 2-stündigem Rühren wurde das Lösungsmittel im Vakuum abgedampft, der Rückstand wurde in 200 ml Essigester aufgenommen, mit Wasser gewaschen und die organische Phase nach Trocknung über Magnesiumsulfat eingedampft.

Der Rückstand lieferte nach Umkristallisieren aus Ethanol 9,6 g N-(4-Carbmethyoxybenzyl)-(5,6,7,8-tetrahydro-5,5,8,8-tetramethyl-2-naphthalenyl)amin, Schmp. 104-107 °C. 2 g des so erhaltenen Esters wurden in 45 ml Ethanol/Dimethylsulfoxid/Wasser (5:1:3) mit 2,5 g Kaliumhydroxid 1 Stunde unter Rückfluß erhitzt. Die Lösung wurde bei Raumtemperatur mit 20 ml 2 N Salzsäure versetzt. Der Niederschlag wurde abfiltriert, aus Methanol umkristallisiert und lieferte 1,1 g der Titelverbindung, Schmp. 225-231 °C.

Beispiel 12

N-Acetyl-N-(4-carboxybenzyl)-(5,6,7,8-tetrahydro-5,5,8,8-tetra-methyl-2-naphthalenyl)amin

4,2 g (12 mmol) N-(4-Carbmethoxybenzyl)-(5,6,7,8-tetrahydro-5,5,8,8-tetramethyl-2-naphthal enyl)amin (aus Beispiel 11) wurden in 100 ml Dichlormethan mit 1,6 ml (16 mmol) Acetanhydrid und 2,3 ml (17 mmol) Triethylamin bei Raumtemperatur gerührt. Nach beendeter Reaktion wurde die Lösung auf Wasser gegossen, mit Essigester extrahiert, die organische Phase abgetrennt und eingedampft; der Rückstand wurde aus Ethanol umkristallisiert und lieferte N-Acetyl-N-(4-carbmethoxybenzyl)-(5,6,7,8-tetrahydro-5,5,8,8-tetramethyl-2-naphthalenyl)amin. 2,5 g dieses Produktes wurden anschließend in 90 ml Ethanol/Wasser/Dimethylsulfoxid (5:3:1) mit 5,0 g Kaliumhydroxid 1 Stunde unter Rückfluß erhitzt, nach dem Abkühlen mit dem 3-fachen Volumen Eiswasser verdünnt und mit 2 N Salzsäure auf pH 4 gestellt. Der Niederschlag wurde abfiltriert und erbrachte nach Trocknung 1,5 g der Titelverbindung, Schmp. 219-223 °C.

Beispiel 13

N-Methyl-N-(4-carboxybenzyl)-(5,6,7,8-tetrahydro-5,5,8,8-tetramethyl-2-naphthalenyl)amin

4,2 g (12 mmol) N-(4-Carbmethoxybenzyl)-(5,6,7,8-tetrahydro-5,5,8,8-tetramethyl-2-naphthalenyl)amin (aus Beispiel 11) wurden mit 2,0 g (30 mmol) Paraformaldehyd in 100 ml Methanol/Tetrahydrofuran (1:1) mit 1,25 g (20 mmol) Natriumcyanoborhydrid versetzt, über Nacht bei Raumtemperatur und anschließend 2 Stunden bei 40 °C gerührt. Nach Abdampfen des Lösungsmittels wurde der Rückstand in Essigester aufgenommen, mit Wasser gewaschen, über Magnesiumsulfat getrocknet und eingedampft.

Der erhaltene Rückstand wurde in 90 ml Ethanol/Wasser/Dimethylsulfoxid (5:3:1) mit 5 g Kaliumhydroxid 1 Stunde unter Rückfluß erhitzt. Die Reaktionslösung wurde bei Raumtemperatur mit 2 N Salzsäure auf pH 5 gestellt. Der resultierende Niederschlag wurde abfiltriert und erbrachte nach umkristallisation aus Ethanol 2,5 g der Titelverbindung, Schmp. 141-144 °C.

Beispiel 14

(4-Carboxybenzyl)-(5,6,7,8-tetrahydro-5,5,8,8-tetramethyl-2-naphthalenyl)sulfoxid

5,3 g (15 mmol) des nach Beispiel 10 erhaltenen Thioethers wurden in einem Gemisch aus 150 ml Ethanol und 40 ml Dimethylformamid bei 0 ° C mit einer Lösung aus 3,4 g (16 mmol) Natriumperiodat in 30 ml Wasser tropfenweise versetzt und 2 Stunden bei gleicher Temperatur gerührt. Anschließend wurde über Nacht bei Raumtemperatur gerührt. Die Reaktionslösung wurde dann auf Wasser gegossen, mit 2 N Salzsäure auf pH 5 gestellt, mit Dichlormethan extrahiert und die organischen Extrakte nach Waschen mit Wasser über Magnesiumsulfat getrocknet und im Vakuum eingedampft. Der ölige Rückstand lieferte aus Ethanol 2,2 g kristalliner Titelverbindung, Schmp. 198-200 ° C.

Beispiel 15

(4-Carboxybenzyl)-(5,6,7,8-tetrahydro-5,5,8,8-tetramethyl-2-naphthalenyl)sulfon

6,3 g (18 mmol) des nach Beispiel 10 erhaltenen Thioethers wurden in 60 ml Eisessig bei 50 ° C tropfenweise mit 6,2 g (54 mmol) Wasserstoffperoxydlösung (30 %ig in Wasser) versetzt. Die Lösung wurde 1 Stunde bei 75 ° C nachgerührt, das Reaktionsprodukt mit Wasser ausgefällt und der Niederschlag abgesaugt.

Umkristallisieren aus Ethanol lieferte 1,6 g der Titelverbindung, Schmp. 202-205 ° C.

Analog wurden die Substanzen der nachfolgenden Tabelle hergestellt.

Tabelle 1:

| Bsp.-Nr. | E | R | A | Schmp. (°C) |
|---|---|---|---|---|
| 16 | H | $CO_2H$ | $-CH_2N(COCH_3)-$ | 228-230 |
| 17 | H | $CO_2H$ | $-CH_2NH-$ | 193-195 |
| 18 | H | $CO_2H$ | $-CH_2S-$ | 197-199 |
| 19 | H | $CO_2H$ | $-CH(CH_3)S-$ | 172-175 |
| 20 | H | $CO_2H$ | $-SCH(CH_3)-$ | 138-140 |
| 21 | H | $CO_2H$ | $-CH_2N(CH_3)-$ | 253-258 |
| 22 | H | $CO_2H$ | $-NHCH(CH_3)-$ | 205-208 |
| 23 | OH | $CO_2H$ | $-CH_2NH-$ | 234-236 |
| 24 | H | $CO_2H$ | $-SCH(CH_3)-$ | 181-185 |
| 25 | H | $NHCOCH_3$ | $-NHCH_2-$ | 192-195 |
| 26 | H | $SCH_3$ | $-NHCH_2-$ | 83-85 |
| 27 | H | $SO_2CH_3$ | $-CH_2O-$ | 158-160 |
| 28 | H | $N(CH_3)_2$ | $-NHCH_2-$ | 113-115 |
| 29 | H | $SCH_3$ | $-CH_2O-$ | 91-94 |
| 30 | H | $NHCOCH_3$ | $-CH_2S-$ | 115-117 |
| 31 | H | $NHCOCH_3$ | $-CH_2O-$ | 126-128 |
| 32 | H | $SOC_2H_5$ | $-NHCH_2-$ | 154-155 |
| 33 | H | $SO_2C_2H_5$ | $-NHCH_2-$ | 140-143 |

Beispiele für pharmazeutische Zubereitungen:

Beispiel I

Tablette mit 250 mg Wirkstoff

| Zusammensetzung für 1000 Tabletten: | |
|---|---|
| Wirkstoff des Beispiels Nr. 2: | 250 g |
| Kartoffelstärke: | 100 g |
| Milchzucker: | 50 g |
| Gelatinelösung 4 %: | 45 g |
| Talkum: | 10 g |

Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel II

| Creme aus 0,1 % Wirkstoff | |
|---|---|
| Wirkstoff des Beispiels Nr. 10: | 0,1 g |
| Glycerinmonostearat: | 10,0 g |
| Cetylalkohol: | 4,0 g |
| Polyethylenglykol-400-stearat: | 10,0 g |
| Polyethylenglykol-sorbitanmonostearat: | 10,0 g |
| Propylenglykol: | 6,0 g |
| p-Hydroxybenzoesäuremethylester: | 0,2 g |
| Entmineralisiertes Wasser: | ad 100,0 g |

Herstellung:

Der feinst gepulverte Wirkstoff wird in 1,2-Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In dieser Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel III

| Puder mit 0,1 % Wirkstoff | |
|---|---|
| Wirkstoff des Beispiels Nr. 11: | 0,1 g |
| Zinkoxid: | 10,0 g |
| Magnesiumoxid: | 10,0 g |
| Hochdisperses Siliciumdioxid: | 2,5 g |
| Magnesiumstearat: | 1,0 g |
| Talkum: | 74,5 g |

Herstellung:

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

## Patentansprüche

**1.** Diphenylheteroalkylderivate der Formel I

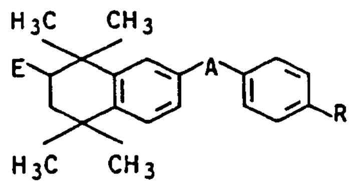

(I)

in der bedeuten:
A eine -X-$CH_2$- oder

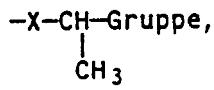

wobei X mit dem linken oder dem rechten Phenylkern verknüpft sein kann und für ein Sauerstoffatom, eine -$S(O)_n$- oder -NR'-Gruppe steht (mit n in der Bedeutung von 0, 1 oder 2 und R' in der Bedeutung von Wasserstoff, $C_{1-6}$-Alkyl oder $C_{1-6}$-Alkanoyl),
E Wasserstoff oder Hydroxyl und
R eine der Gruppen -$CO_2H$, -$CO_2$-$C_{1-6}$-Alkyl, -CHO, -$CH_2OH$, -CN, -$CONH_2$, -$CH_2NH_2$, -NH-$C_{1-4}$-Alkanoyl, -N($C_{1-4}$-Alkyl)$_2$, -$S(O)_n$-$C_{1-4}$-Alkyl (mit n = 0, 1 oder 2),
sowie deren physiologisch verträgliche Salze.

**2.** Verfahren zur Herstellung der Diphenylheteroalkylderivate der Formel I gemäß Anspruch 1, dadurch gekennzeichnet, daß man
a) - falls A eine -O-$CH_2$- oder -O-CH($CH_3$)-Gruppe bedeutet - Phenole der Formel II

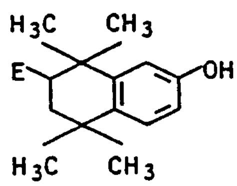

(II),

mit Benzylderivaten der Formel III

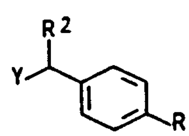

(III),

in der $R^2$ ein Wasserstoffatom oder eine Methylgruppe und Y eine nukleofuge Abgangsgruppe darstellt, in Gegenwart einer Base umsetzt, oder
b) - falls A eine -$CH_2$-O- oder -CH($CH_3$)-O-Gruppe bedeutet - Phenole der Formel IV

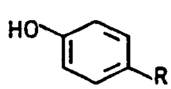

(IV),

mit Benzylderivaten der Formel V

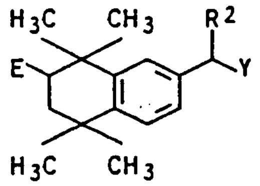

(V),

in der E, $R^2$ und Y jeweils die genannten Bedeutungen haben, in Gegenwart einer Base umsetzt, oder

c) - falls A eine $-S(O)_n-CH_2-$ oder $-S(O)_n-CH(CH_3)$-Gruppe bedeutet - Thiophenole der Formel VI

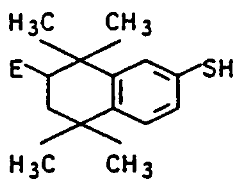

(VI),

mit Benzylderivaten der Formel III wie unter a) angegeben zu Thioethern (n = 0) umsetzt und diese gegebenenfalls mit einem Oxidationsmittel in die korrespondierenden Sulfoxide (n = 1) oder Sulfone (n = 2) überführt, oder

d) - falls A eine $-CH_2-S(O)_n-$ oder $-CH(CH_3)-S(O)_n$-Gruppe bedeutet -Thiophenole der Formel VII

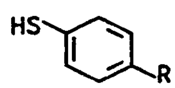

(VII),

in der R die in Anspruch 1 angegebene Bedeutung hat, mit Benzylderivaten der Formel V wie unter b) angegeben zu Thioethern (n = 0) umsetzt und diese gegebenenfalls mit einem Oxidationsmittel in die korrespondierenden Sulfoxide (n = 1) oder Sulfone (n = 2) überführt, oder

e) - falls A eine $-NR'CH_2-$ oder $-NR'CH(CH_3)$-Gruppe bedeutet - entweder

Anilinderivate der Formel VIII

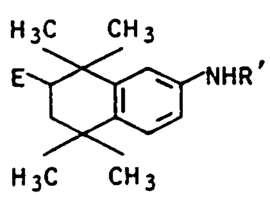

(VIII),

mit Benzylderivaten der Formel III wie unter a) angegeben umsetzt, oder

Anilinderivate der Formel VIII mit Carbonylverbindungen der Formel IX

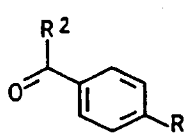

(IX),

in der R und $R^2$ die genannten Bedeutungen haben, in Gegenwart eines Reduktionsmittels umsetzt, oder

f) - falls A eine $-CH_2NR'-$ oder $-CH(CH_3)NR'$-Gruppe bedeutet - entweder

Anilinderivate der Formel X

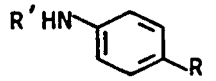

(X),

mit Benzylderivaten der Formel V wie unter b) angegeben umsetzt, oder
Aniline der Formel X mit Carbonylverbindungen der Formel XI

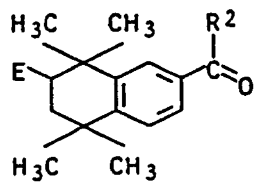

(XI),

in Gegenwart eines Reduktionsmittels umsetzt
und die so erhaltenen Verbindungen gegebenenfalls nach Standardmethoden in weitere Verbindungen der Formel I und gegebenenfalls ihre physiologisch verträglichen Salze umwandelt.

**3.** Arzneimittel zur topischen Anwendung, das als Wirkstoff 0,001 bis 1 Gew.-% eines der Diphenylheteroalkylderivate der Formel I gemäß Anspruch 1 neben üblichen galenischen Hilfsstoffen enthält.

**4.** Arzneimittel zur systemischen Anwendung, das neben üblichen galenischen Hilfsmitteln als Wirkstoff pro Einzeldosis 0,1 bis 250 mg eines der Diphenylheteroalkylderivate der Formel I gemäß Anspruch 1 neben üblichen galenischen Hilfsstoffen enthält.

**5.** Kosmetische Zubereitung, die neben üblichen kosmetischen Hilfsstoffen 0,001 bis 1 Gew.-% eines der Diphenylheteroalkylderivate der Formel I gemäß Anspruch 1 enthält.

**6.** Arzneimittel oder Kosmetikum nach Anspruch 3 oder 5 zur Behandlung von Akne oder Psoriasis oder anderen mit pathologisch veränderter Verhornung einhergehenden dermatologischen Erkrankungen sowie von Ekzemen, Warzen, Vitiligo, von durch UV-Licht oder iatrogen bedingten Schädigungen der Haut sowie von Präkanzerosen und Tumoren und trockenen Augen und anderen Corneopathien.

**7.** Arzneimittel nach Anspruch 4 zur Behandlung von rheumatischen und arthritischen Erkrankungen.

**Claims**

**1.** A diphenylheteroalkyl derivative of the formula I

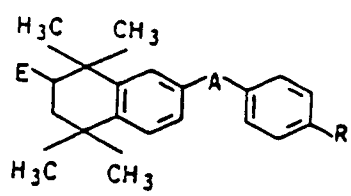

(I)

where
A is $-X-CH_2-$ or

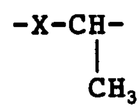

where X can be linked to the left or right phenyl nucleus and is oxygen, $-S(O)_n-$ or $-NR^1-$ (with n being

0, 1 or 2 and $R^1$ being hydrogen, $C_{1-6}$-alkyl or $C_{1-6}$-alkanoyl),
E is hydrogen or hydroxyl and
R is one of $-CO_2H$, $-CO_2-C_{1-6}$-alkyl, -CHO, $-CH_2OH$, -CN, $-CONH_2$, $-CH_2NH_2$, -NH-$C_{1-4}$-alkanoyl, -N-($C_{1-4}$-alkyl)$_2$ or -S(O)$_n$-$C_{1-4}$-alkyl (n being 0, 1 or 2), and the physiologically tolerated salts thereof.

**2.** A process for the preparation of a diphenylheteroalkyl derivative of the formula I as claimed in claim 1, which comprises

a) if A is $-O-CH_2-$ or $-O-CH(CH_3)-$, reacting a phenol of the formula II

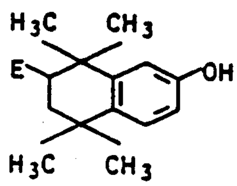

(II),

with a benzyl derivative of the formula III

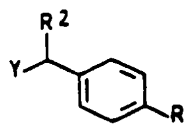

(III),

where $R^2$ is hydrogen or methyl, and Y is a nucleofugic leaving group, in the presence of a base, or

b) if A is $-CH_2-O-$ or $-CH(CH_3)-O-$, reacting a phenol of the formula IV

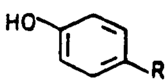

(IV),

with a benzyl derivative of the formula V

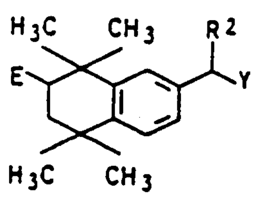

(V),

where E, $R^2$ and Y each have the meanings mentioned, in the presence of a base, or

c) if A is -S(O)$_n$-$CH_2$- or -S(O)$_n$-$CH(CH_3)$-, reacting a thiophenol of the formula VI

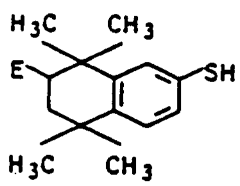

(VI),

with a benzyl derivative of the formula III as specified under a) to give a thioether (n = 0) and possibly converting the latter with an oxidizing agent into the corresponding sulfoxide (n = 1) or sulfone (n = 2), or

d) if A is $-CH_2-S(O)_n-$ or $-CH(CH_3)-S(O)_n-$, reacting a thiophenol of the formula VII

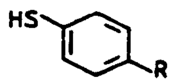

(VII),

where R has the meaning specified in claim 1, with a benzyl derivative of the formula V as specified under b) to give a thioether (n = 0), and possibly converting the latter with an oxidizing agent into the corresponding sulfoxide (n = 1) or sulfone (n = 2), or

e) if A is $-NR'CH_2-$ or $-NR'CH(CH_3)-$, either reacting an aniline derivative of the formula VIII

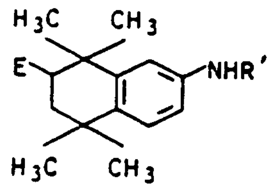

(VIII),

with a benzyl derivative of the formula III as specified under a), or

reacting an aniline derivative of the formula VIII with a carbonyl compound of the formula IX

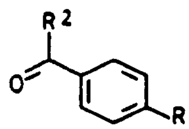

(IX),

where R and $R^2$ have the meanings mentioned, in the presence of a reducing agent, or

f) if A is $-CH_2NR'-$ or $-CH(CH_3)NR'-$, either reacting an aniline derivative of the formula X

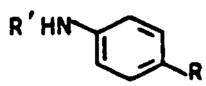

(X),

with a benzyl derivative of the formula V as specified under b), or

reacting an aniline of the formula X with a carbonyl compound of the formula XI

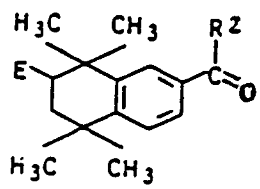

(XI),

in the presence of a reducing agent,

and possibly converting the resulting compounds by standard methods into other compounds of the formula I or into their physiologically tolerated salts.

**3.** A drug for topical use which contains as active substance 0.001 to 1 % by weight of a diphenylheteroalkyl derivative of the formula I as claimed in claim 1 in addition to conventional pharmaceutical auxiliaries.

**4.** A drug for systemic use which contains as active substance 0.1 to 250 mg of a diphenylheteroalkyl derivative of the formula I as claimed in claim 1 per single dose, in addition to conventional

pharmaceutical auxiliaries.

5. A cosmetic preparation which contains 0.001 to 1 % by weight of a diphenylheteroalkyl derivative of the formula I as claimed in claim 1 in addition to conventional cosmetic auxiliaries.

6. A drug or cosmetic as claimed in claim 3 or 5 for the treatment of acne or psoriasis or other dermatological disorders associated with pathological keratinization as well as of eczema, warts, vitiligo, UV-induced or iatrogenic damage to the skin and of precanceroses and tumors and dry eyes and other corneopathies.

7. A drug as claimed in claim 4 for the treatment of rheumatic and arthritic disorders.

**Revendications**

1. Dérivés diphénylhétéroalkyliques de la formule I

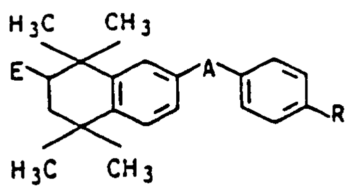

(I)

dans laquelle
A représente un radical $-X-CH_2-$ ou

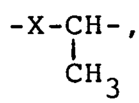

où X peut être au noyau phénylique gauche ou droit et représente un atome d'oxygène, un radical $-S(O)_n-$ ou -NR'- (n étant égal à 0, 1 ou 2 et R' représentant un atome d'hydrogène, un radical alkyle en $C_1$-$C_6$ ou alcanoyle en $C_1$-$C_6$),
E représente un atome d'hydrogène ou le radical hydroxyle et
R représente l'un des radicaux qui suivent : $-CO_2H$, $-CO_2$-alkyle-$C_1$-$C_6$, -CHO, $-CH_2OH$, -CN, $-CONH_2$, $-CH_2NH_2$,-NH-alcanoyle-$C_1$-$C_4$, -N(alkyle-$C_1$-$C_4$), $-S(O)_n$-alkyle-$C_1$-$C_4$ (n = 0, 1 ou 2),
ainsi que leurs sels physiologiquement compatibles.

2. Procédé de préparation de dérivés diphénylhétéroalkyliques de la formule I suivant la revendication 1, caractérisé en ce que
a) dans le cas où A représente un groupe $-O-CH_2-$ ou $-O-CH(CH_3)-$, on fait réagir des phénols de la formule II

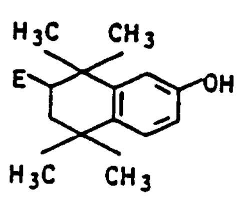

(II)

avec des dérivés benzyliques de la formule III

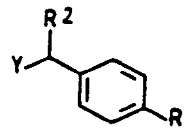

(III)

dans laquelle $R^2$ représente un atome d'hydrogène ou le radical méthyle et Y représente un groupe sortant nucléofuge, en présence d'une base, ou
b) au cas où A représente un radical $-CH_2-O-$ ou $-CH(CH_3)-O-$, on fait réagir des phénols de la formule IV

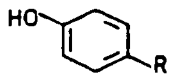

(IV)

avec des dérivés benzyliques de la formule V

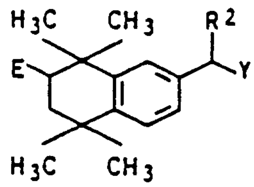

(V)

dans laquelle E, $R^2$ et Y possèdent chacun les significations qui leur ont été précédemment attribuées, en présence d'une base, ou
c) au cas où A représente un radical $-S(O)_n-CH_2-$ ou $-S(O)_n-CH(CH_3)-$, on convertit des thiophénols de la formule VI

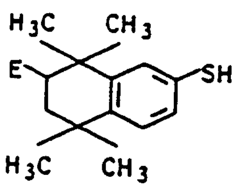

(VI)

avec des dérivés benzyliques de la formule III tels que définis sous a) en thioéthers (n = 0) et on transforme ceux-ci en les sulfoxydes correspondants (n = 1) ou en les sulfones correspondantes (n = 2), éventuellement à l'aide d'un agent d'oxydation, ou
d) au cas où A représente un radical $-CH_2-S(O)_n-$ ou $-CH(CH_3)-S(O)_n-$, on convertit des thiophénols de la formule VII

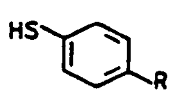

(VII)

dans laquelle R possède les significations qui lui ont été attribuées dans la revendication 1, avec des dérivés benzyliques de la formule V tels que définis sous b), en thioéthers (n = 0) et on transforme ces derniers en les sulfoxydes correspondants (n = 1) ou les sulfones correspondantes (n = 2), éventuellement à l'aide d'un agent d'oxydation, ou
e) au cas où A représente un radical $-NR'CH_2-$ ou $-NR'CH(CH_3)-$, on fait réagir des dérivés de l'aniline de la formule VIII

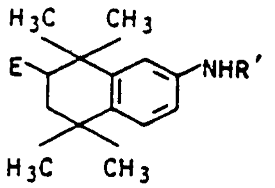

(VIII)

avec des dérivés benzyliques de la formule III tels que définis sous a), ou on fait réagir des dérivés de l'aniline de la formule VIII avec des composés carbonylés de la formule IX

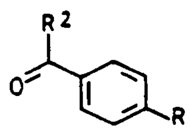

(IX)

dans laquelle R et $R^2$ possèdent les significations qui leur ont été précédemment attribuées, en présence d'un agent réducteur, ou
f) au cas où A représente un radical $-CH_2NR'-$ ou $-CH(CH_3)NR'-$, on fait réagir des dérivés de l'aniline de la formule X

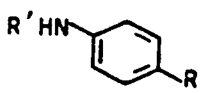

(X) ,

avec des dérivés benzyliques de la formule V tels que définis sous b), ou on fait réagir des anilines de la formule X avec des composés carbonylés de la formule XI

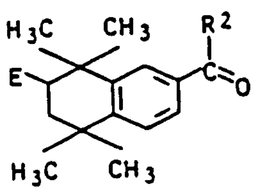

(XI)

en présence d'un agent réducteur
et on transforme les composés ainsi obtenus en d'autres composés de la formule I, éventuellement selon des méthodes standard et on les convertit éventuellement en leurs sels physiologiquement compatibles.

3. Médicament destiné à l'utilisation topique, qui contient, à titre de principe actif, 0,001 à 1% en poids d'un dérivé diphénylhétéroalkylique de la formule I selon la revendication 1, outre les excipients ou adjuvants galéniques habituels.

4. Médicament destiné à l'utilisation systémique, qui contient, outre les adjuvants ou excipients galéniques usuels, à titre de principe actif et par dose unitaire, 0,1 à 250 mg d'un dérivé diphénylhétéroalkylique de la formule I selon la revendication 1.

5. Préparation cosmétique, qui contient, outre les adjuvants cosmétiques usuels, de 0,001 à 1% en poids d'un dérivé diphénylhétéroalkylique de la formule I selon la revendication 1.

6. Médicament ou cosmétique selon la revendication 3 ou 5, destiné au traitement de l'acné, du psoriasis et d'autres maladies dermatologiques associées à une kératinisation pathologiquement modifiée et

contre des dommages cutanés déclenchés par la lumière ultraviolette ou à cause iatrogène, comme aussi pour le traitement d'eczémas, de verrues, du vitiligo, de tumeurs et d'états précancéreux, des yeux secs et d'autres cornéopathies.

**7.** Médicament suivant la revendication 4, destiné au traitement de maladies rhumatismales et arthritiques.